# Europäisches Patentamt

# European Patent Office

# Office européen des brevets

(19)

(11) Veröffentlichungsnummer: **0 102 538**
**B1**

(12) **EUROPÄISCHE PATENTSCHRIFT**

(45) Veröffentlichungstag der Patentschrift:
06.08.86

(21) Anmeldenummer: **83107741.7**

(22) Anmeldetag: **05.08.83**

(51) Int. Cl.⁴: **A 61 M 25/00**, A 61 B 17/38

(54) **Punktier- und Katheterisiervorrichtung für menschliche oder tierische Körper.**

(30) Priorität: **06.08.82 DE 3229466**

(43) Veröffentlichungstag der Anmeldung:
**14.03.84 Patentblatt 84/11**

(45) Bekanntmachung des Hinweises auf die Patenterteilung:
**06.08.86 Patentblatt 86/32**

(84) Benannte Vertragsstaaten:
**AT BE CH DE FR GB IT LI LU NL SE**

(56) Entgegenhaltungen:
**US - A - 3 547 103**
**US - A - 3 682 162**
**US - A - 3 698 394**
**US - A - 4 128 173**
**US - A - 4 205 675**
**US - A - 4 269 174**

(73) Patentinhaber: **Sterimed Gesellschaft für medizinischen Bedarf mbH, Fasanerieweg 15-17 Postfach 215, D-6600 Saarbrücken 3 (DE)**

(72) Erfinder: **März, Peter, Dr., Wankstrasse 5, D-8000 München 70 (DE)**
Erfinder: **Postel, Jürgen, Dr., Feldmochinger Strasse 26, D-8000 München 50 (DE)**

(74) Vertreter: **Hoene, Dieter, Dr., c/o Byk Gulden Lomberg Chemische Fabrik GmbH Postfach 6500 Byk-Gulden-Strasse 2, D-7750 Konstanz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

### Technisches Gebiet

Die Erfindung bezieht sich auf eine Punktier- und Katheterisiervorrichtung für menschliche und tierische Körper mit einer metallischen Punktiernadel und einer die Punktiernadel in dem Bereich zwischen deren Spitze und rückwärtigem Ende umgebenden Kanüle, durch welche nach Herausziehen der Punktiernadel ein längerer Führungsdraht in den Körper eingeführt werden kann, der dann nach Herausziehen auch der Kanüle als Führung für einen zu legenden Katheter dient.

### Stand der Technik

Vorrichtungen dieser Art sind als Katheter für Körpergefässe, wie z. B. Venen oder Körperhöhlen, wie z. B. die Bauchhöhle, bekannt. So ist in der US-A-4128173 ein Katheterset und dessen Anwendung zur Behandlung der Bauchhöhle beschrieben. Zuerst wird die Bauchhöhle mit einer Kanüle, in der sich eine Punktiernadel befindet, punktiert. Die Punktiernadel wird aus der Kanüle gezogen und ein Führungsdraht durch die Kanüle in die Bauchhöhle geschoben. Die Kanüle wird von dem Führungsdraht abgezogen. Nachdem ein Katheterschlauch über den Führungsdraht bis an den gewünschten Zielort in der Bauchhöhle vorgeschoben ist, wird der Führungsdraht entfernt. Durch den Katheterschlauch kann nunmehr beispielsweise der Bauchhöhle Flüssigkeit zugeführt oder Flüssigkeit aus ihr entnommen werden.

Die bekannten Vorrichtungen sind aber nicht zum Punktieren und Katheterisieren von Nervensträngen geeignet, da es bei einem solchen Vorgang sehr schwer ist, die Punktiernadel richtig in das Innere einer Nervenscheide bzw. in den Zwischenraum zwischen Nervenscheide und Nerv einzuführen.

Aus der US-A-3682162 ist eine Punktiernadel bekannt, an deren Spitze zwei Elektroden enden. Den Elektroden können über elektrische Anschlüsse am rückwärtigen Ende der Punktiernadel elektrische Impulse aus einem Impulsgeber zugeführt werden. Die Nadel dient dazu, mit Hilfe der durch die elektrischen Impulse ausgelösten Muskelkontraktionen bestimmte Nerven aufzusuchen und diese dann durch Einspritzen eines Lokalanästhetikums durch die Punktiernadel zu betäuben. Vorrichtungen dieser Art gestatten aber nur ein unmittelbares Einspritzen von Substanzen durch die Punktiernadel, ohne dass eine Möglichkeit gegeben wäre, nun nach Einführung der Punktiernadel in den Zwischenraum zwischen Nervenscheide und Nerv einen längeren Katheter einzuführen und für längere Dauer zu legen.

Der vorliegenden Erfindung liegt die Aufgabe zugrunde, bei einer Vorrichtung der in Rede stehenden Art Vorkehrungen zu treffen, um diese zum Punktieren und Katheterisieren von Nervensträngen brauchbar zu machen.

Die Lösung der Aufgabe wird dadurch erreicht, dass die Punktiernadel von ihrer Spitze bis zu ihrem aus der Kanüle herausragenden Ende elektrisch leitend ausgeführt und am rückwärtigen Ende mit einem elektrischen Anschlussteil ausgestattet ist.

Bei einer derartigen Ausgestaltung der Vorrichtung kann man das Legen von Nervenkathetern wesentlich vereinfachen. An das elektrische Anschlussteil der Punktiernadel lässt sich ein elektrischer Impulsgeber anschliessen, wie er im Handel unter der Bezeichnung Neurotracer erhältlich ist, und damit wird es möglich, sich mit der Punktiernadel und der sie umgebenden Kanüle genau im Inneren der Nervenscheide voranzutasten und unerwünschte Abweichungen sofort festzustellen. Wenn eine unerwünschte Abweichung auftritt, erreichen die eingegebenen Stromimpulse den Nerv nicht mehr, und der behandelnde Arzt kann dann an der Reaktion des Patienten sofort feststellen, dass er die Nadel anders führen muss.

Fig. 1 zeigt in vergrössertem Massstab eine Punktier- und Katheterisiervorrichtung für den menschlichen Körper mit elektrischem Anschlussteil zum Anschluss eines elektrischen Impulsgebers; dabei sind die Punktiernadel und die sie umgebende Kanüle abgebrochen gezeichnet;

Fig. 1A veranschaulicht schematisch ein herausgebrochen gezeichnetes Stück der Kanüle der Vorrichtung aus Fig. 1 mit einem hindurchgeführten Führungsdraht (Mandrin), der zum Legen eines Nervenkatheters dienen kann;

Fig. 1B veranschaulicht schematisch und ebenfalls in vergrössertem Massstab ein Stück eines mittels des Führungsdrahtes in den menschlichen Körper einzuführenden Nervenkatheters;

Fig. 2 zeigt schematisch eine Punktier- und Katheterisiervorrichtung der in Fig. 1 gezeigten Art mit anderer Ausführung des elektrischen Anschlussteils und einem Faltenschlauch zur sterilen Abdeckung der elektrischen Anschlussleitung.

Die in den Zeichnungen gezeigte Punktier- und Katheterisiervorrichtung dient zur Herstellung einer Verbindung des einen Nerv aufnehmenden Innenraums einer Nervenscheide mit der Umgebung. Bei der medizinischen Behandlung wird eine derartige Verbindung gebraucht, um beispielsweise den Innenraum der Nervenscheide zu katheterisieren, Behandlungsmittel zu infundieren o. dgl. Für diesen Zweck müssen gelegentlich auch längere Katheter an tiefer im Körper gelegene Stellen eingeführt werden, ähnlich, wie es beim Legen von Venenkathetern der Fall ist.

Die gezeigte Punktier- und Katheterisiervorrichtung hat eine mit einer Spitze 1 versehene Punktiernadel 3, welche beispielsweise 60 mm lang ist und wie die Nadel einer Injektionsspritze zum Einstechen in den Körper geeignet ist.

Die Punktiernadel besteht aus elektrisch leitfähigem Material und hat an ihrem rückwärtigen Teil ein noch näher zu beschreibendes elektrisches Anschlussteil 5, welches mit einer elektrischen Anschlussleitung 7 ausgestattet ist.

Mittels der elektrischen Anschlussleitung 7 lässt sich die Punktiernadel 3 an einen nicht näher dargestellten elektrischen Impulsgeber anschliessen, wie er beispielsweise im Handel unter der Bezeichnung «Neurotracer» erhältlich ist. Die von dem elektrischen Impulsgeber gegebenen Impulse ermöglichen es dem behandelnden Arzt, sich mit der Punktiernadel 3 im Inneren der Nervenscheide voranzubewegen. Sobald die Punktiernadel 3 mit der sie umgebenden isolierenden Kanüle 9 in den menschlichen Körper eingestochen worden ist und in eine Nervenscheide eingedrungen ist, können die eingeleiteten Stromimpulse auf den Nerv wirksam werden und führen dann zu einer äusserlich sichtbaren Bewegungsreaktion entsprechender Körperteile, beispielsweise der Hand.

Solange die Punktiernadel in der Nervenscheide bleibt, bleiben die über die Punktiernadel eingegebenen elektrischen Impulse wirksam, um die Bewegungsreaktionen des Körperteils auszulösen. Solange diese Bewegungsreaktionen sichtbar sind, weiss der behandelnde Arzt, dass er die Punktiernadel und mit ihr die Kanüle richtig in dem Zwischenraum zwischen Nerv und Nervenscheide entlangführt.

Dabei braucht die Punktiernadel im übrigen nur ein kurzes Stück in der Nervenscheide entlanggeführt zu werden und gibt dann bereits eine ausreichende Führung für die Kanüle 9, die dann mittels des Handhabungsansatzes 11 gegenüber der Punktiernadel 3 in der Nervenscheide vorangeschoben werden kann.

Die Kanüle 9 hat in der angedeuteten Weise eine etwas geringere Länge als die Punktiernadel 3, so dass deren Spitze 1 aus der isolierenden Kanüle 9 nach vorne heraussteht. Die isolierende Kanüle besteht aus einem geeigneten Kunststoffmaterial und ist auch entsprechend schmal ausgeführt, um ein Einführen in eine Nervenscheide zu ermöglichen.

Um das zunächst erforderliche gemeinsame Einführen von Punktiernadel 3 und Kanüle 9 zu ermöglichen, ist ein Handhabungsansatz 13 am rückwärtigen Ende der Punktiernadel 3 bei 15 teleskopartig mit einer Muffe 17 am rückwärtigen Ende der Kanüle 9 zusammengesteckt, wobei diese Steckverbindung leicht lösbar ist, um die Kanüle 9 in der angegebenen Weise gegenüber der Punktiernadel 3 voranzuschieben.

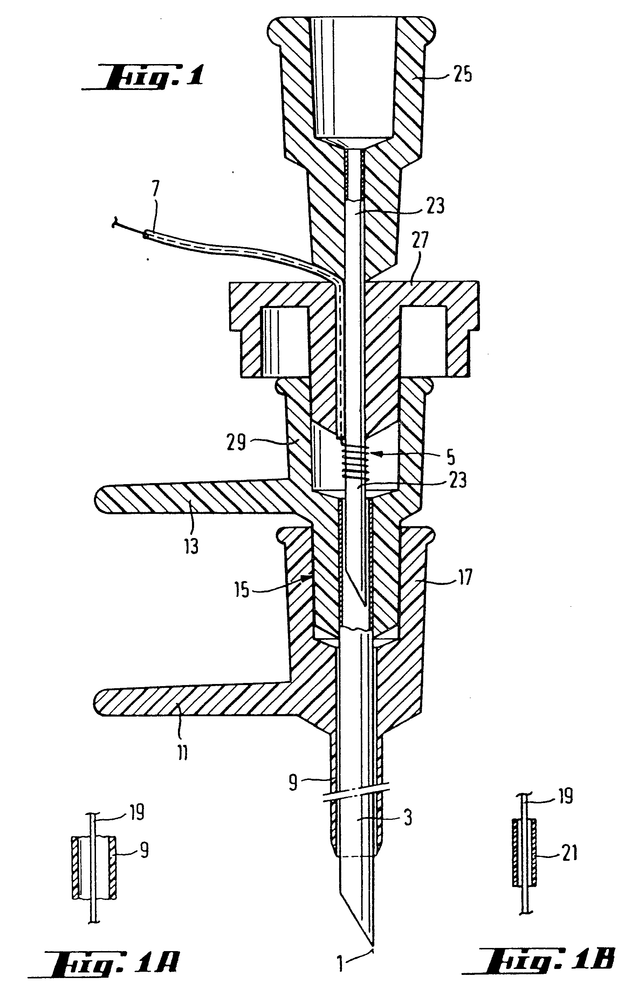

Die Muffe 17 der Kanüle 9 dient nach Herausziehen der Punktiernadel 3 bei der weiteren Behandlung aber auch zum Einführen eines in Fig. 1A schematisch innerhalb der Kanüle 9 gezeigten Führungsdrahts 19, dem sogenannten Mandrin, der dann nach Herausziehen auch der Kanüle 9 aus dem Körper in der in Fig. 1B schematisch angedeuteten Weise zum Einführen eines Nervenkatheters 21 dienen kann. Führungsdraht 19 und Nervenkatheter 21 haben beispielsweise eine Länge von 25 cm.

Das in Fig. 1 gezeigte elektrische Anschlussteil hat eine metallische Nadel 23, welche in das rückwärtige Ende der Punktiernadel 3 eingesteckt ist und damit in elektrisch leitender Verbindung steht. (Der gezeichnete Zwischenraum dient lediglich der besseren Darstellbarkeit der Nadeln, stellt aber keineswegs einen isolierenden Zwischenraum dar.)

Die metallische Nadel 23 kann selbst auch als Hohlnadel ausgeführt und mit einem rückwärtigen Anschlussstutzen 25 versehen sein, um selbst bei hergestelltem elektrischem Anschluss Injektionen o. dgl. vornehmen zu können.

Die metallische Nadel 23 ist von einem Anschlussstopfen 27 umgeben, der mit einer Anschlussbuchse 29 am rückwärtigen Handhabungsteil 13 der Punktiernadel 3 zusammensteckbar ist. Dabei dient der Anschlussstopfen 27 zugleich zur Befestigung der elektrischen Anschlussleitung 7, die zu dem elektrischen Impulsgeber führt.

Es versteht sich von selbst, dass der männliche Konus des Anschlussstopfens 27 dichtend in den weiblichen Konus der Anschlussbuchse 29 eingepasst ist, damit während des Punktiervorganges mit am Anschlussstutzen 25 aufgesetzter Injektionsspritze auch angesaugt werden kann.

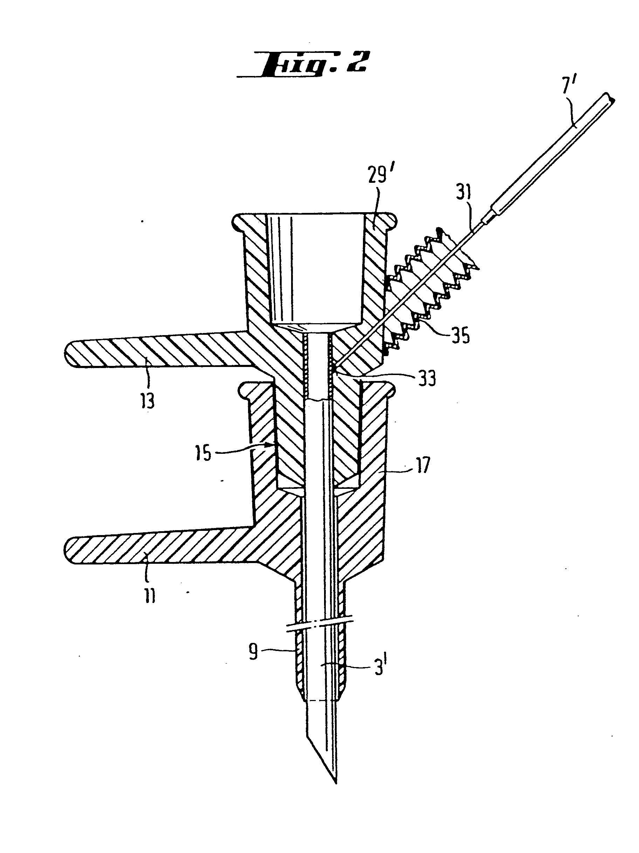

Wie aus Fig. 2 ersichtlich, kann ein elektrischer Anschluss für das rückwärtige Ende der Punktiernadel 3' auch einfach dadurch hergestellt werden, dass durch die Anschlussbuchse 29' der Punktiernadel seitlich ein elektrischer Leiter 31 eingeführt ist, der bei 33 in elektrischer Verbindung mit der Punktiernadel steht. Auch auf diese Weise lässt sich über die Leitung 7' ein einfacher Anschluss an einen elektrischen Impulsgeber herstellen.

Die Fig. 2 veranschaulicht schematisch auch die Anordnung eines Faltenschlauchs 35, der den aus der Anschlussbuchse 29 austretenden elektrischen Leiter 31 und insbesondere auch dessen Austrittsstelle abgedichtet umgeben kann. Das eröffnet die Möglichkeit, die elektrischen Anschlussteile, wie den Leiter 31 und die Leitung 7' an der Vorrichtung selbst kurz zu halten und statt dessen bei entsprechend langer Ausgestaltung des Faltenschlauchs 35 die von dem elektrischen Impulsgeber herführende Leitung steril bis dicht an die Punktier- und Katheterisiervorrichtung heranzuführen. Diese Vorrichtung lässt sich ja steril als Massenteil herstellen und bei Benutzung braucht dann nur der Faltenschlauch 35 über die von dem elektrischen Impulsgeber herkommende Leitung gezogen zu werden, um die Sterilität der Umgebung durch diese Leitung nicht zu gefährden.

Das elektrische Anschlussteil am rückwärtigen Ende der Punktier- und Katheterisiervorrichtung lässt sich auch auf verschiedene andere Weisen verwirklichen, die hier nicht näher dargestellt und beschrieben sind.

So kann der elektrische Anschluss beispielsweise mittels einer sogenannten Krokodilklemme an einem dafür freiliegend vorgesehenen Stück am rückwärtigen Ende der Punktiernadel hergestellt werden.

### Patentansprüche

1. Punktier- und Katheterisiervorrichtung für menschliche oder tierische Körper mit einer metallischen Punktiernadel (3; 3') und einer die Punktiernadel (3; 3') in dem Bereich zwischen deren Spitze (1) und rückwärtigem Ende umgebenden Kanüle (9), durch welche nach Herausziehen der Punktiernadel (3; 3') ein längerer Führungsdraht (19) in den Körper eingeführt werden kann, der dann nach Herausziehen auch der Kanüle (9) als Führung für einen zu legenden Katheter (21) dient, dadurch gekennzeichnet, dass die Punktiernadel (3; 3') von ihrer Spitze (1) bis zu ihrem aus der Kanüle (9) herausragenden Ende elektrisch leitend ausgeführt und am rückwärtigen Ende mit einem elektrischen Anschlussteil (5; 31, 33) ausgestattet ist.

2. Vorrichtung nach Anspruch 1, dadurch gekennzeichnet, dass die Punktiernadel (3; 3'), wie an sich bekannt, hohl ausgestaltet und das elektrische Anschlussteil (5) am rückwärtigen Ende derart ausgebildet ist, dass ein unbehinderter Flüssigkeitsstrom durch die Punktiernadel (3; 3') möglich ist.

3. Vorrichtung nach Anspruch 2, dadurch gekennzeichnet, dass das elektrische Anschlussteil (5; 31, 33) von einer in das rückwärtige Ende der Punktiernadel (3; 3') einsteckbaren hohlen metallischen Nadel (23) gebildet wird, mit der eine elektrische Anschlussleitung (7) verbunden ist.

4. Vorrichtung nach einem oder mehreren der voranstehenden Ansprüche, dadurch gekennzeichnet, dass die Austrittsstelle der elektrischen Leitung (31) am rückwärtigen Ende der Punktiernadel (3; 3') von einem Faltenschlauch (35) umgeben ist, der über eine von aussen heranführbare elektrische Leitung (7') ausziehbar ist.

## Claims

1. A puncturing and catheterizing device for the human or animal body with a metallic puncture needle (3; 3') and a cannula (9) which surrounds the puncture needle (3; 3') in the region between its point (1) and rear end and through which, after the puncture needle (3; 3') has been pulled out, a longer guidewire (19) can be inserted into the body, which then, after the cannula (9) has also been pulled out, serves as a guide for a catheter (21) to be inserted, characterized in that the puncture needle (3; 3') is electrically conductive from its point (1) to its end projecting out of the cannula (9) and is equipped with an electrical connecting piece (5; 31, 33) at the rear end.

2. A device as claimed in claim 1, characterized in that the puncture needle (3; 3'), as known per se, is hollow and the electrical connecting piece (5) at the rear end is designed such that an unimpeded flow of liquid through the puncture needle (3; 3') is possible.

3. A device as claimed in claim 2, characterized in that the electrical connecting piece (5; 31, 33) is formed from a hollow metallic needle (23) which can be inserted into the rear end of the puncture needle (3; 3') and to which an electrical connecting lead (7) can be connected.

4. A device as claimed in any one of the preceding claims, characterized in that the exit site of the electrical lead (31) at the rear end of the puncture needle (3; 3') is surrounded by a flexible tube (35) which can be drawn over an electrical lead (7') brought up from outside.

## Revendications

1. Dispositif de ponction et de cathétérisme pour le corps humain ou le corps d'un animal, comportant une aiguille de ponction métallique (3; 3') et une canule (9) entourant l'aiguille de ponction (3; 3') dans l'intervalle compris entre sa pointe (1) et l'extrémité postérieure, par laquelle on peut introduire dans le corps, après avoir retiré l'aiguille de ponction (3; 3'), un fil de guidage (19) plus long, qui sert alors, après que l'on ait retiré également la canule (9), de guidage pour un cathéter (21) à poser, caractérisé en ce que l'aiguille de ponction (3; 3') est réalisée de façon à être électriquement conductrice de sa pointe (1) à son extrémité sortant de la canule (9), et comporte, à son extrémité postérieure, un élément de raccordement électrique (5; 31, 33).

2. Dispositif selon la revendication 1, caractérisé en ce que, de façon en soi connue, l'aiguille de ponction (3; 3') est creuse, et en ce que l'élément de raccordement électrique (5) situé à l'extrémité postérieure est conformé de façon à permettre un écoulement de liquide sans obstacle à travers l'aiguille de ponction (3; 3').

3. Dispositif selon la revendication 2, caractérisé en ce que l'élément de raccordement électrique (5; 31, 33) est formé par une aiguille métallique creuse (23) pouvant être insérée dans l'extrémité postérieure de l'aiguille de ponction (3; 3') et à laquelle est relié un conducteur de raccordement électrique (7).

4. Dispositif selon une ou plusieurs des revendications précédentes, caractérisé en ce que le point de sortie du conducteur électrique (31) est entouré, à l'extrémité postérieure de l'aiguille de ponction (3; 3') par un tube en accordéon (35) que l'on peut étirer sur un conducteur électrique (7') pouvant être amené de l'extérieur.

0102538

Fig. 1

Fig. 1A

Fig. 1B

0102538

# *Fig. 2*